# EUROPEAN PATENT APPLICATION

(11) **EP 3 428 609 A1**
(43) Date of publication of application: **16.01.2019**
(21) Application number: 17841260.7
(22) Date of filing: 19.05.2017
(51) Int. Cl.: G01N 1/10, A61B 5/15, G01N 1/12, B04B 5/02, G01N 33/48

(54) **BLOOD SAMPLER AND BLOOD SAMPLING TOOL COMPRISING BLOOD CONTAINER**

(30) Priority: 17.08.2016 JP 2016160250
(71) Applicant: Micro Blood Science Inc., Tokyo 1010032 (JP)
(72) Inventor: IWASAWA Hajime, Tokyo 101-0032 (JP); NISHIMURA Tomoaki, Tokyo 101-0032 (JP); NEMOTO Shota, Tokyo 101-0032 (JP)
(74) Representative: Parchmann, Stefanie
(86) International application number: PCT/JP2017/018763
(87) International publication number: WO 2018/034032

(57) **Abstract**

The object of the present invention is to provide a blood sampling tool with which, even when a small amount of sample is collected, the small amount of sample can be collected in a state suitable for the later examination. The blood sampling tool of the present invention comprises; a pipette tip-shaped blood sampler having openings at an upper part and a lower part having an interior space for aspirating and retaining blood by the capillary action; a blood container having a hydrophilically treated inner wall and bottom and having a protrusion provided at the bottom that is able to accommodate the blood collected by the blood sampler; and a sealing lid for sealing the blood container. With this blood sampling tool, when the lower opening of the blood sampler having aspirated and retained blood in the interior space is brought into contact with the inner wall or bottom of the blood container, the blood transfers easily from the blood sampler into the blood container.

## Description

### [Technical Field]

The present invention relates to a blood sampling tool comprising a blood sampler and a blood container for collecting a small amount of blood.

### [Background Art]

It is known that condition changes and diseases in the living body appear in changes in components of body fluid, for example, blood, urine, saliva and the like as biological reactions. Then, a change in physical condition is monitored and diseases are detected, by measuring and analyzing components contained in body fluid.

Particularly, blood tests measuring blood components are widely conducted as an effective means for investigating diseases and as a means for health management.

When measuring blood components, it is required to reliably collect the necessary amount of blood, particularly peripheral blood. Then, the collected blood is transferred into a blood container and then subjected to analysis according to a predetermined purpose.

Further, a blood collection kit comprises a blood collecting tool and a blood collecting bottle is suggested (Patent document 1). This blood collecting tool is composed of a cylindrical liquid absorbing unit for performing temporal liquid absorption disposed at the end and containing blood coagulation preventing liquid and a pushing unit which pushes the rear surface of the liquid absorbing unit to separate the liquid absorbing unit, while, a chemical solution is contained in the blood collecting bottle. When the blood collecting tool is pushed into the blood collecting bottle and then the pushing unit of the blood collecting tool is pushed, blood is eluted from the end of the blood collecting tool into the chemical solution. Since the collected blood is mixed in the chemical solution, however, plasma of the collected blood cannot be separated, and additionally, the amount of a specific component in the blood cannot be measured correctly.

Further, a blood collector for small amount of blood using a disposable pipette composed of a dropper tube and a flexible cap in communication has been proposed (Patent Document 2). In this blood collector, liquid is absorbed temporarily, then, the absorbed liquid can be released by compressing or blowing a gas.

Likewise, there is a suggestion on a body fluid sampling apparatus composed of a body fluid sampler equipped with an aspirating part for aspirating body fluid, a body part having a ventilation passage for allowing a gas to pass therethrough for ejecting the body fluid aspirated by the aspirating part and a blocking part provided between the body part and the aspirating part; and a body fluid container capable of accommodating the body fluid that has been collected by the body fluid sampler (Patent Document 3). Here, the body fluid aspirated by the aspirating part of the body fluid sampler is transferred from the aspirating part into the body fluid container by pressure generated by a pressure generation part connected to the body part.

The blood test using the collected blood is performed using an automatic analyzer, but it is necessary to transfer the blood collected using the blood sampler into the blood container.

However, in the conventional sampling apparatus as described above, pressure is used to eject the aspirated body fluid, there is a possibility of generation of problems such as hemolysis due to the force applied upon release. Also, if pursuing the convenience, it is preferable that the vessel itself to be used transfers blood into a blood container.

### [Citation List]

### [Patent Literature]

[Patent Literature 1] Japan Unexamined Patent Application Publication No. 2006-288680
[Patent Literature 2] Japan Unexamined Patent Application Publication No. 2005-017281
[Patent Literature 3] WO 2015/034009

### [Summary of the Invention]

### [Technical Problem]

In view of the above problems, it is a purpose of the present invention to provide a blood sampling tool excellent in convenience, and even when a small amount of sample is collected, the small amount of sample can be collected in a state suitable for the later examination.

### [Solution to Problem]

The blood sampling tool of the present invention is a blood sampling tool comprising; a pipette tip-shaped blood sampler having openings at an upper part and a lower part having an interior space for aspirating and retaining blood by the capillary action; a blood container having a hydrophilically treated inner wall and bottom that is able to accommodate the blood that has been collected by the blood sampler; and a sealing lid for sealing the blood container. When the lower opening of the blood sampler having aspirated and retained blood in the interior space is brought into contact with the inner wall or bottom of the blood container, the blood transfers easily from the blood sampler into the blood container.

### [Advantageous Effect of the Invention]

According to the present invention, blood can be easily transferred from a blood sampler into a blood container without using a special discharge mechanism. Also, since extra force and pressure are not applied when discharging blood, the collected blood can be accommodated in the blood container without occurrence of problems such as hemolysis. Such blood is suitable for the subsequent blood analysis.

### [Brief Description of Drawings]

Fig. 1 is a view showing the configuration of a first embodiment of a blood sampling tool according to the present invention.
Fig. 2 is an explanatory view of one embodiment of collecting blood using a blood sampler of a blood sampling tool according to the present invention.
Fig. 3 is a view explaining the transfer of blood from a blood sampler into a blood container in a blood sampling tool of the first embodiment of the present invention.
Fig. 4 is a view explaining another mode of blood transfer from a blood sampler into a blood container in a blood sampling tool of the first embodiment of the present invention.
Fig. 5 is a view showing the configuration of a second embodiment of a blood sampling tool according to the present invention.

### [Description of Embodiments]

The invention will now be described in conjunction with preferred methods and materials which can be used in the practice of the invention, by way of exemplary embodiments.

Unless otherwise specified in the text, all technical terms and scientific terms used in present specification have the same meaning as commonly understood by one of ordinary skill in the art to which this invention belongs. Also, any materials and methods equivalent or similar to those described in present specification can be used as well in the practice of the present invention.

Further, all publications and patents cited in present specification in connection with the invention described herein constitute a part of present specification, for example, as referring to methods and materials and the like that can be used in the present invention.

Hereinafter, a blood sampling tool which is an embodiment of the present invention will be explained with reference to drawings.

### (First Embodiment)

A first embodiment of the blood sampling tool according to the present invention is
a blood sampling tool comprising;
a pipette tip-shaped blood sampler having openings at an upper part and a lower part having an interior space for aspirating and retaining blood, wherein the lower opening and the upper opening communicate in the interior space of the blood sampler, at least a part of the interior space has a tapered shape from the lower opening toward the upper opening, and it is configured that when the lower opening is brought into contact with blood, the blood is aspirated into the interior space by the capillary action;
a blood container that is able to accommodate the blood that has been collected by the blood sampler, wherein the blood container has a space inside into which the blood sampler can be inserted and has a hydrophilically treated inner wall and bottom; and
a sealing lid for sealing the blood container;
wherein it is configured that when the lower opening of the blood sampler having aspirated and retained blood in the interior space is brought into contact with the inner wall or bottom of the blood container, the blood transfers from the blood sampler into the blood container.

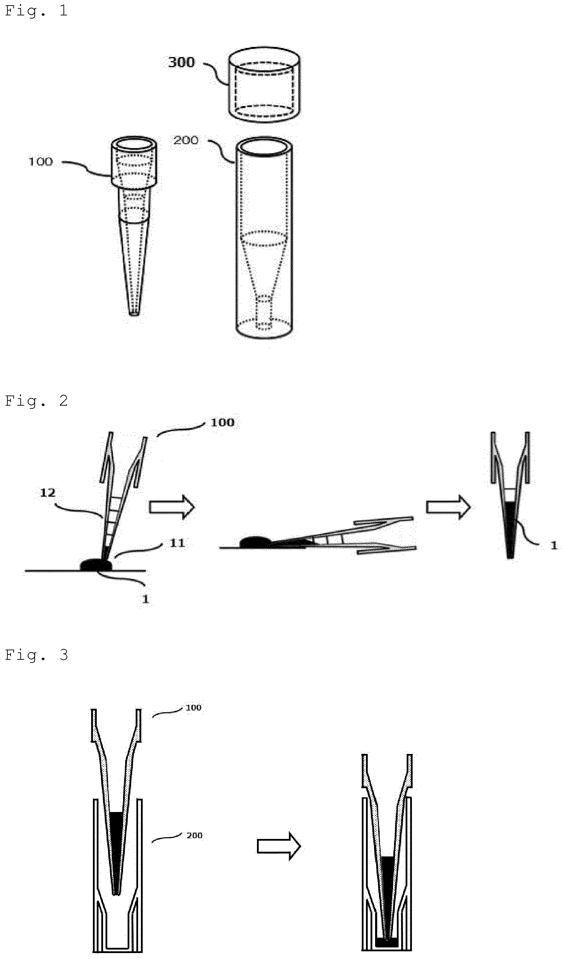

Fig. 1 shows a first embodiment of the present invention. The blood sampling tool of the present invention comprises a blood sampler 100, a blood container 200 and a sealing lid 300 for sealing the blood container.

As shown in Fig. 2, when the lower opening 11 of the blood sampler 100 is brought into contact with blood 1, the blood is aspirated into the interior space of the blood sampler 100 by the capillary action. If the blood sampler is tilted to aid in aspiration, blood can be aspirated more easily. In the drawing, a scale 12 for checking the amount of blood is shown, but the scale may or may not be attached.

For example, when collecting peripheral blood from a fingertip or an earlobe, blood can be collected by puncturing a fingertip or an earlobe and then bringing the lower opening of the blood sampler into contact with the blood that has come out, thereby inducing the blood into the blood sampler by the capillary action or negative pressure generated by flow of fluid.

In collection of blood, there is no limit to the blood collection amount, and the blood amount can be varied according to the subsequent purpose. With the blood sampler of the present invention, any small amount of blood (for example, any amount from 10 µL up to 200 µL) can be collected.

Fig. 3 is a view showing a blood sampler retaining blood, the sampler being inserted into a blood container, for transferring the aspirated blood into the blood container. As shown on the right view, when the lower opening of the blood sampler is brought into contact with the bottom of the hydrophilically treated blood container, the blood transfers easily into the blood container.

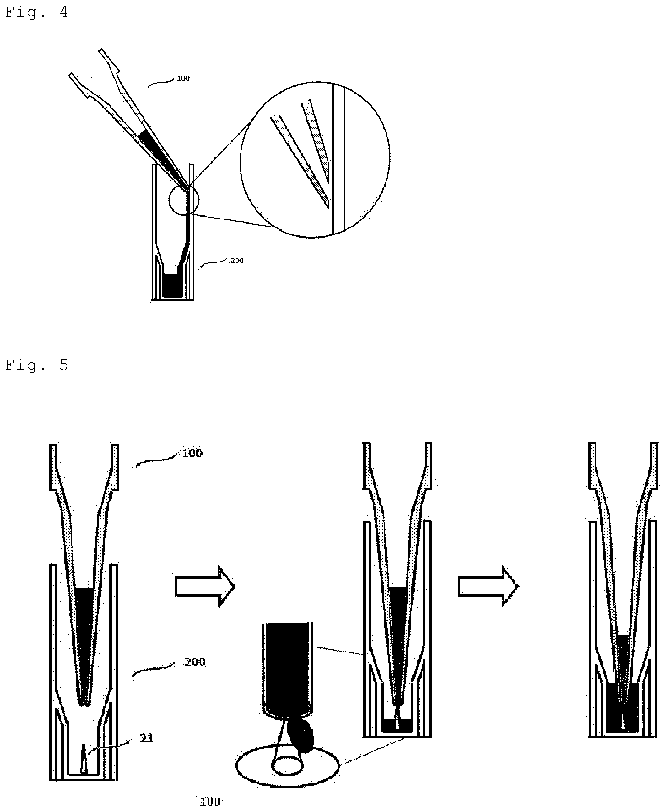

As shown in Fig. 4, the lower opening of the blood sampler retaining the blood aspirated may be brought into contact with the hydrophilically treated side wall of the blood container. Even in such cases, blood is easily transferred from the blood sampler into the blood container by gravity and hydrophilic action. As shown in an enlarged view in Fig. 4, it is also possible to process the opening of the tip end of the blood sampler obliquely.

In the blood sampling tool according to the present invention, after collecting blood using the blood sampler, the blood transfers from the blood sampler into the blood container easily by bringing the lower opening of the blood sampler into contact with the inner wall or bottom of the blood container. Before bringing the lower opening of the blood sampler into contact with the inner wall or bottom of the blood container, the blood aspirated by the capillary action is retained in the blood sampler, but if it is brought into contact with the hydrophilically treated inner wall or bottom, the blood is drawn by gravity and hydrophilic action, and it transfers into the blood container. That is, when the lower opening of the blood sampler in a state that blood is retained in the blood retaining portion is brought into contact with the inner wall or bottom of the blood container, the blood aspirating force (X) based on the capillary action of the blood sampler and the blood discharging force (Y) based on the gravity of the blood and the hydrophilic treatment of the blood container satisfy the relation: X<Y, thereby the blood transfers from the blood sampler into the blood container. Particular when the inner wall of the blood retaining portion of the blood sampler is hydrophilically treated to facilitate collection of blood, movement of blood into the blood container is insufficient only by gravity and the hydrophilic treatment of the inner wall and bottom of the blood container is essential.

In the step of transferring blood into the blood container using the tool of the present invention, force such as air pressure or the like for discharging the blood in the blood sampler is unnecessary and there is no concern of causing problems such as hemolysis and the like.

### (Second Embodiment)

A second embodiment of the blood sampling tool according to the present invention is the blood sampling tool of the first embodiment in which a protrusion is formed on the bottom of the blood container. The step of transferring blood from the blood sampler into the blood container using the blood sampling tool of the second embodiment of the present invention is shown in Fig. 5. Since the bottom including the protrusion is hydrophilically treated, when the blood in the vicinity of the lower opening of the blood sampler is brought into contact with the protrusion, the blood is discharged and easily transferred into the blood container.

In the blood sampling tool of the present invention, after blood has been transferred into the blood container, the blood container can be sealed by the sealing lid. In that state, transport and storage of the blood is possible. In addition, it is also possible to centrifuge blood in a state where the blood is accommodated in the blood container, thus, the blood can be separated into the supernatant (serum or plasma) and the deposit (blood cell) .

The hydrophilic treatment of the inner wall and the bottom of the blood container can be carried out by a known method and can be attained by, for example, but not limited to, etching, fluorine treatment, or formation of irregularities and fine scratches on the surface. As examples, it is possible to impart hydrophilicity by forming porous parts on the inner wall surface by chemical treatments such as chemical vapor deposition, or by introducing a hydrophilic functional group on the inner wall surface via a catalyst. Treatments for imparting hydrophilicity to the surface of a material composed of a polymer compound, for example, polypropylene or polycarbonate are known and these methods can be used. For example, a method using a mixed gas of a fluorine gas and an oxygen gas and a technology described in Hidehiko Enomoto, Toshiya Murata, "Hyomen Gijyutsu" (Surface technology), Vol. 59, No. 5, p. 282 - 287 (2008) can be used without limitation to them.

A portion for retaining blood after collection of the blood sampler 100 of the present invention (referred to as "blood retaining portion" in the present specification) has a similar shape of a sample aspirating portion of a pipette tip whose inner diameter is continuously increased. The capacity of the blood retaining portion is preferably 200 µL or less, more preferably 150 µL or less, more preferably 100 µL or less.

The inner diameter of the lower end opening of the blood sampler 100 and the inner diameter of the fuselage part which is the blood retaining portion can be arbitrarily determined as long as the effect of the capillary action can be obtained. The inner diameter of the lower end opening of the blood sampler 100 is, for example, but not limited to, 0.1 mm or more and less than 2.0 mm, preferably 0.3 mm or more and less than 1.5 mm, more preferably 0.5 mm or more and less than 1.0 mm, further preferably 0.7 mm or more and less than 0.9 mm. This makes it easy to collect blood from the lower opening by the capillary action.

It is preferable that the blood sampler is composed of a polymer material such as polyvinyl chloride, polyethylene, polypropylene, acrylonitrile butadiene styrene (ABS), polycarbonate, polyethylene terephthalate and the like, from the standpoint of workability and operability.

The above exemplified materials are water repellent. Therefore, in the tip of a micropipette intending discharge after aspirating a solution, since it has separate power for aspirating and discharging, the hydrophobicity of the surface of the chip material causes high drainage when discharging the aspirated solution. However, when using the capillary action to aspirate blood, this property is not preferred, thus, in the blood sampler in the tool of the present invention, the inner wall of the blood retaining portion is preferably hydrophilically treated. When the inner wall is not hydrophilically treated, a problem occurs that blood is less likely to flow into smoothly when collecting the blood. In the blood sampler of the present invention, the capillary action and the effect of the hydrophilic treatment of the inner wall are combined to allow easy collection of a target amount of blood.

The hydrophilic treatment of the inner wall and the bottom of the blood container can be carried out by a known method and can be attained by, for example, but not limited to, etching, fluorine treatment, or formation of irregularities and fine scratches on the surface. As examples, it is possible to impart hydrophilicity by forming porous parts on the inner wall surface by chemical treatments such as chemical vapor deposition, or by introducing a hydrophilic functional group on the inner wall surface via a catalyst. Treatments for imparting hydrophilicity to the surface of a material composed of a polymer compound, for example, polypropylene or polycarbonate are known and these methods can be used. For example, a method using a mixed gas of a fluorine gas and an oxygen gas and a technology described in Hidehiko Enomoto, Toshiya Murata, "Hyomen Gijyutsu" (Surface technology), Vol. 59, No. 5, p. 282 - 287 (2008) can be used without limitation to them.

The blood container 200 is not limited in its shape and is not limited in its material as long as the blood sampler 100 can be inserted and its lower opening can be brought into contact with the wall surface or bottom of the container. Considering the sample collection by the operator, graspability, transportation, etc., a hard material which has high transparency is preferable. The sealing lid 300 of the blood container 200 is not limited in its shape and its material as long as the lid can seal the container, and it is preferable that the sealing lid is capable of preventing drying of the internal sample and elution at the time of centrifugation.

The amount of blood collected by the blood sampler can be changed according to the intended examination and measurement, and the length and diameter of the sampler can be adjusted accordingly. The amount of the sample to be collected can be adjusted within a range of, for example, but not limited to, 10 µL to 300 µL, preferably 10 µL to 200 µL, more preferably 50 µL to 100 µL.

Further, by providing the blood sampler with the guide line 12 indicating that a specific amount has been collected, it is possible to accurately collect a specific amount of blood.

By using the blood sampling tool of the present invention, it is possible to prepare good blood suitable for blood analysis even with a small amount of peripheral blood collected from a fingertip or an earlobe. Such blood, by using diluted, can be analyzed according to conventional protocols and existing analyzers used for the analysis of brachial vein blood collected by a conventional blood collection tube or the like. That is, using a small amount of peripheral blood collected from the fingertip, ear lobe or the like, the same blood analysis as before can be performed.

For example, the blood collected using the blood sampling tool of the present invention can be used in examinations of general biochemical items and/or immune items, so-called blood biochemical examinations, commonly used in blood examinations, and additionally, also for special examination items such as cancer markers and allergy.

### (Example of hydrophilic treatment of blood container)

### Processing Example 1: Hydrophilic treatment of inner wall and bottom of sampling tool by etching

A molded blood container of ABS was immersed in a solution containing hexavalent chromium for 12 minutes thereby dissolving butadiene on the surface and making etching holes on the surface, to form fine porous parts.

Processing Example 2: Hydrophilic treatment of inner wall and bottom of blood container with mixed gas of fluorine gas and oxygen gas

In a molded blood container of polycarbonate, a hydrophilic treatment by a fluorine gas treatment was entrusted to Takamatsu Teisan Corporation (Takamatsu, Kagawa Prefecture) based on Japanese Unexamined Patent Application Publication No. 2010-150460, and a surface modification treatment was conducted using a mixed gas of a fluorine gas and an oxygen gas at a fluorine gas partial pressure of 1.33 Pa and an oxygen gas partial pressure of 93100 Pa at a treatment temperature of 25°C for a treatment time of 600 seconds.

In this fluorine treatment, hydrophilicity of a carboxyl group, a hydroxyl group or the like can be developed by utilizing high reactivity of a fluorine gas and oxygen.

### Processing Example 3: Hydrophilic treatment of inner wall and bottom of blood container by surfactant coating

The following was performed using the blood sampling tool of the present invention shown in Fig. 5.

In a molded blood container of polycarbonate and having a protrusion, the inner wall and the bottom were immersed in a 0.2% (w/w) polyoxyethylene sorbitan monolaurate aqueous solution for 30 seconds, then, washed with pure water and dried well, thereby performing a hydrophilic treatment by surfactant coating.

When the tip of the blood sampler having aspirated 50 µL of the colored solution was brought into contact with the protrusion of the blood container, the solution moved immediately into the blood container. In contrast, the same procedure was conducted on the blood sampler not subjected to the above-described hydrophilic treatment, but the solution did not move.

The above description merely illustrates the purpose and objects of the present invention and is not intended to limit the scope of the appended claims. Without departing from the scope of the appended claims, various alterations and substitutions for the described embodiments will be apparent to those skilled in the art from the teachings described in the present specification.

### [Explanation of numerals]

1. blood
11. lower opening
12. guide line
21. protrusion
100. blood sampler
200. blood container
300. sealing lid

## Claims

1. A blood sampling tool comprising;
a pipette tip-shaped blood sampler having openings at an upper part and a lower part having an interior space for aspirating and retaining blood, wherein the lower opening and the upper opening communicate in the interior space of the blood sampler, the lower portion of the interior space constituting a blood retaining portion having a hydrophilically treated inner wall has a tapered shape from the lower opening tip toward the upper opening, and it is configured that when the lower opening is brought into contact with blood, the blood is aspirated into the interior space by the capillary action;
a blood container that is able to accommodate the blood collected by the blood sampler, wherein the blood container has a space inside into which the blood sampler can be inserted and has a hydrophilically treated inner wall and bottom; and
a sealing lid for sealing the blood container;
wherein an protrusion is provided at the bottom of the blood container and the blood retained in the blood retaining portion is brought into contact with the protrusion, thereby discharging the blood into the blood container.

2. A blood sampling tool comprising;
a pipette tip-shaped blood sampler having openings at an upper part and a lower part having with an interior space for aspirating and retaining blood, wherein the lower opening and the upper opening communicate in the interior space of the blood sampler, the lower portion of the interior space constituting a blood retaining portion having a hydrophilically treated inner wall has a tapered shape from the lower opening tip toward the upper opening, and it is configured that when the lower opening is brought into contact with blood, the blood is aspirated into the interior space by the capillary action;
a blood container that is able to accommodate the blood collected by the blood sampler, wherein the blood container has a space inside into which the blood sampler can be inserted and has a hydrophilically treated inner wall and bottom; and
a sealing lid for sealing the blood container;
wherein it is configured that when the lower opening of the blood sampler in a state that blood is retained in the blood retaining portion is brought into contact with the inner wall or bottom of the blood container, the blood aspirating force (X) based on the capillary action of the blood sampler and the blood discharging force (Y) based on the gravity of the blood and the hydrophilic treatment of the blood container satisfy the following relation: X<Y, and an protrusion is provided at the bottom of the blood container and the blood retained in the blood retaining portion is brought into contact with the protrusion, thereby discharging the blood into the blood container.

3. The blood sampling tool according to Claim 1 or 2, wherein the hydrophilic treatment of the blood sampler or the blood container is performed by etching or fluorine processing.

4. The blood sampling tool according to Claim 1 or 2, wherein the hydrophilic treatment of the blood container is performed by forming irregularities or small scratches on the surface of the inner wall or bottom of the blood container.

5. The blood sampling tool according to any one of Claims 1 to 4, wherein the lower opening is an opening having an inner diameter of 0.5 mm or more and less than 1.0 mm.
